# EUROPEAN PATENT APPLICATION

(11) **EP 4 524 249 A1**
(43) Date of publication of application: **19.03.2025**
(21) Application number: 23796356.6
(22) Date of filing: 25.04.2023
(51) Int. Cl.: C12N 15/45, C07K 14/115, C12N 1/15, C12N 1/19, C12N 1/21, C12N 5/10, C12N 7/01, C12N 15/63, C12N 15/86

(54) **RNA VIRUS-DERIVED CHIMERIC ENVELOPE PROTEIN AND RNA VIRUS VECTOR HAVING SAME**

(30) Priority: 25.04.2022 JP 2022071680
(71) Applicant: Tokiwa-bio Inc., Tsukuba-shi, Ibaraki 305-0047 (JP)
(72) Inventor: NAKANISHI, Mahito, Tsukuba-shi, Ibaraki 305-0047 (JP); MIZUTA, Shiori, Tsukuba-shi, Ibaraki 305-0047 (JP)
(74) Representative: Kraus & Lederer PartGmbB
(86) International application number: PCT/JP2023/016214
(87) International publication number: WO 2023/210616

(57) **Abstract**

The present invention addresses the problem of providing a chimeric envelope protein that pseudotypes a virus, and also providing efficient gene transfer and gene expression techniques to lymphocytes such as B cells, CD4 positive T cells, and CD8 positive T cells contained in peripheral blood and immortalized cells derived from these cells, said techniques being characterized by using an RNA virus vector having the aforesaid chimeric protein. In a gene transfer method using a single-stranded RNA virus vector such as a Sendai virus vector or a stealth RNA vector, the virus is pseudotyped by using, as the envelope proteins of viral particles, a chimeric F protein having a morbillivirus-derived F protein region and a chimeric H protein having a morbillivirus-derived H protein region.

## Description

### Technical Field

The present invention relates to technology for transferring and expressing exogenous genes into animal cells.

### Background Art

A Sendai virus belonging to the Genus *Respirovirus* of *Paramyxoviridae* has an outer membrane (envelope) composed of a lipid bilayer membrane and a glycoprotein, and is a non-segmented, negative-sense, single-stranded RNA virus in which genomic RNA is sent into the cytoplasm of a host cell by the fusion of the envelope and the cell membrane, and gene expression is performed in a cytoplasm (Non-Patent Document 1). Since the Sendai virus does not have pathogenicity, genotoxicity, or tumorigenicity to humans, the Sendai virus is highly safe, and can be mass-produced using hatched chicken eggs. Therefore, the Sendai virus has been widely used in industrial applications such as production of interferon and research applications as a tool of cell fusion.

In recent years, recombinant Sendai virus vectors carrying exogenous gene(s), and vectors constructed by synthetic biology techniques based on hints from the Sendai virus have been used in various industrial applications. For example, a defective persistent expression type Sendai virus (SeVdp) vector (Patent Document 1 and Non-Patent Document 2) prepared using a Sendai virus Clone 151 strain as a material has been used for producing induced pluripotent stem cells (iPS cells) by carrying exogenous genes encoding 4 reprogramming factors in one genome (Patent Document 2). A recombinant Sendai virus vector carrying a fibroblast growth factor 2 (FGF2) gene has been used in clinical trials of gene therapy for inducing vascular regeneration to treat critical limb ischemia (Patent Document 3, Non-Patent Document 3). A stealth RNA vector (SRV) reconstituted from a nucleic acid having artificial sequences optimized for human cells using the activity of the RNA-dependent RNA polymerase of paramyxovirus, has lower cytotoxicity as compared with a Sendai virus vector, completely lacks the ability of autonomous replication, and can stably express genetic information in a cytoplasm at a physiological level (Patent Document 4). Therefore, SRV is expected to be applied as an industrial tool for gene transfer and expression, such as gene therapy, regenerative medicine, and biopharmaceutical production, as well as conventional retrovirus vectors, lentivirus vectors, and adeno-associated virus vectors (AAV).

One of the advantages of the recombinant Sendai virus vector is that it has a wide range of host and cell specificities, and can transfer and express genes into cells of various animal species. It is known that the Sendai virus can be fused with cells of many mammals such as monkeys, mice, rats, dogs, rabbits, and hamsters in addition to human cells (Non-Patent Document 4). In the human cells, it has been confirmed that genes can be transferred and expressed in fibroblasts, epithelial cells, neural cells, muscle cells, hepatic cells, multipotent stem cells, hematopoietic stem cells, cartilage cells, and peripheral blood monocytes (Non-Patent Document 5).

The infection of the Sendai virus and the gene transfer by the recombinant Sendai virus vector occur in two stages of (1) binding of virus particles (vector particles) to cell membranes and (2) fusion of virus outer membranes (envelope) and cell membranes (Non-Patent Document 1). This phenomenon is performed by the coordinated action of an HN protein and an F protein present in the envelope of the Sendai virus (Non-Patent Document 1). In the present specification, a virus in which a negative-sense single-stranded RNA genome is packaged in an envelope is referred to as a "virus particle". The RNA genome in the virus particle may or may not have exogenous gene(s). In the present specification, a virus particle in which an RNA genome comprising exogenous gene(s) is packaged may be referred to as a "vector particle". The vector particle is a recombinant virus capable of expressing exogenous gene(s), carried in the genome, in a host cell. In the present specification, the vector particle may be referred to as a "virus vector" or an "RNA virus vector" from the viewpoint of transferring and expressing exogenous gene(s) into a host cell.

The HN protein has sialic acid hydrolase (sialidase) activity, and is involved in the binding of virus particles (vector particles) in the first stage to the cell membrane by binding to sialic acid which is a component of glycolipids and glycoproteins present on the cell membrane (Non-Patent Document 1). Sialic acid is a molecule that is universally present in any animal cell, and this is one of the reasons why a Sendai virus or a recombinant Sendai virus vector has a wide host range.

The F protein is synthesized as a precursor of F₀, and then cleaved by a protein hydrolase to become an active form composed of subunit F₁ and subunit F₂ (Non-Patent Document 1).

In the N-terminal region of the subunit F₁, there is a hydrophobic site in which hydrophobic amino acid residues are continuous, and the characteristic of this hydrophobic site induces the second-stage fusion between the virus membrane and the cell membrane (Non-Patent Document 6).

Lymphocytes (B cells, CD4 positive T cells, CD8 positive T cells, etc.) contained in human peripheral blood are important materials in industrial applications of human cells. For example, CD8-positive T cells have the ability to attack target cells in an immune response, and studies have progressed in which CD8-positive T cells express a chimeric T antigen (CAR-T) that recognizes cancer cells and are used for treatment of cancer (Non-Patent Document 7). The B cells play a role of producing antibodies, and introduction of an immortalization gene by an EB virus is an important means for obtaining a human monoclonal antibody (Non-Patent Document 8). The CD4-positive T cells have a role of regulating the activity of the entire immune system, and studies on the production of regulatory T cells (RegT cells), which suppress an excessive immune reaction in the living body by ectopic expression of the transcription factor Fox3P in the CD4-positive T cells, have attracted attention as a therapeutic method for autoimmune diseases (Non-Patent Document 9). As described above, human peripheral blood lymphocytes are important target cells for which gene transfer is required in industrial applications.

Although the Sendai virus has a wide host range, it is known that infection efficiency with lymphocytes such as B cells, CD4 positive T cells, and CD8 positive T cells in peripheral blood and immortalized cells derived from these cells is exceptionally low (Non-Patent Document 5). Therefore, it was expected that the gene transfer efficiency into B cells, CD4 positive T cells, and CD8 positive T cells was also low in a Sendai virus vector such as the above-described SeVdp vector or a stealth RNA vector which performs gene transfer using the outer membrane glycoprotein of the Sendai virus.

From the analysis of fusion between the virus outer membrane and the cell membrane using intracellular delivery of fragment A of diphtheria toxin as an index, it has been revealed that in B cells, CD4 positive T cells and CD8 positive T cells, which are less likely to be infected with the Sendai virus, the virus particle and the cell membrane normally bind to each other in the initial stage of infection in two stages, but the efficiency of subsequent fusion between the virus outer membrane and the cell membrane is low (Non-Patent Document 10). In addition, similarly, it is known that there is a cell in which the binding between the virus particle and the cell membrane is normal, but the efficiency of subsequent fusion between the virus outer membrane and the cell membrane is low (Non-Patent Document 11). Analysis of these cells suggests the presence of an unknown factor involved in the fusion of the virus outer membrane and the cell membrane, but a responsible molecule has not yet been identified, and a technology to increase the efficiency of gene transfer into lymphocytes using a Sendai virus vector is also not known.

As a measure for improving gene transfer efficiency by modifying the host cell specificity of an enveloped virus having an outer membrane (envelope) composed of a lipid bilayer membrane, a technique called "pseudo-typing" is known in which outer membrane glycoproteins related to infection are substituted with outer membrane glycoproteins derived from other viruses having different host cell specificity. A lentivirus vector using human immunodeficiency virus (HIV) as a material is one example in which host cell specificity can be manipulated by pseudo-typing.

Lentivirus vectors having the outer membrane glycoprotein gp160 encoded by the Env gene of HIV are able to transfer genes into CD4-positive human T cells that bind to gp160, but most human cells are CD4-negative and cannot be used for gene transfer. With respect to the broadening of the host cell specificity of the lentivius vectors, a vesicular stomatitis virus (VSV) pseudotyped vector in which gp160 is substituted with G protein present in the envelope of VSV (Non-Patent Document 12) and a Measles virus (MeV) pseudotyped vector in which gp160 is substituted with an F protein and an H protein present in the envelope of MeV (Non-Patent Document 13) have been reported.

The VSV pseudotyped lentivirus vector is capable of transferring genes into a very wide range of animal cells. In this case, by expressing the G protein of VSV instead of gp160, it is possible to produce a VSV pseudotyped lentivirus vector of 4 x 10⁵ transduction units (tdu)/mL (Non-Patent Document 12).

Meanwhile, the MeV pseudotyped lentivirus vector has a higher gene transfer efficiency into blood cells including B cells, CD4 positive T cells, and CD8 positive T cells than that of the lentivirus vector having gp160. However, in this case, even if the MeV F protein and the H protein are expressed instead of gp160, only a vector with a low concentration of 30 tdu/mL or less can be produced (Non-Patent Document 13). Meanwhile, when the C-terminal 30 amino acid residues of the F protein and the N-terminal 18 amino acid residues of the MeV H protein are deleted in expressing the MeV F protein and the H protein, an MeV pseudotyped vector of 10⁵ tdu/mL can be produced (Non-Patent Document 13). As described above, the modification of the gene transfer efficiency by the pseudotyped virus largely depends on the type and structure of the envelope glycoprotein to be used.

From the findings in the lentivirus vectors, it is conceivable that in a gene transfer method using a Sendai virus vector or a stealth RNA vector, the efficiency of gene transfer into B cells, CD4 positive T cells and CD8 positive T cells could be improved by pseudo-typing the virus vector by replacing the envelope protein of virus particles with an MeV F protein and an H protein in place of the envelope protein derived from the Sendai virus. However, while Sendai virus is included in the Genus *Respirovirus* of the Family *Paramyxoviridae,* there are no reports of technology for pseudo-typing a Sendai virus vector such as a SeVdp vector, or a stealth RNA vector that uses the infection mechanism of the Sendai virus to transfer genes, using an F protein and an H protein of a virus of Genus *Morbillivirus* of the Family *Paramyxoviridae,* including MeV.

In order to produce pseudotyped Sendai virus vectors or stealth RNA vectors, first, conditions under which envelope proteins derived from other virus species are incorporated into virus particles must be clarified. In Non-Patent Document 14, a method for producing a pseudotyped Sendai virus using an envelope protein derived from human parainfluenza virus type 1 (hPIV1) of the same Genus *Respirovirus* as that of the Sendai virus is shown. According to the method, by substituting the cytoplasmic domain of the envelope protein of hPIV1 with the cytoplasmic domain of the envelope protein of the Sendai virus, chimeric envelope proteins of hPIV1 and Sendai virus are incorporated into the Sendai virus particles, thereby obtaining a Sendai virus pseudotyped with hPIV1. However, according to Non-Patent Document 15, it is shown that even when the cytoplasmic domain or transmembrane domain of the envelope protein of MeV is substituted with the corresponding amino acid sequence of the envelope protein of the Sendai virus, this modified envelope proteins are not incorporated into the Sendai virus particles at all. From this, it has been considered that the Sendai virus can be pseudotyped only when an envelope protein derived from the Genus *Respirovirus* is used, and it is impossible to pseudotype the Sendai virus with MeV contained in the Genus *Morbillivirus.*

### Citation List

### Patent Document

Patent Document 1: WO 2008/129971 A1
Patent Document 2: WO 2012/063817 A1
Patent Document 3: WO 2002/42481 A1
Patent Document 4: WO 2016/114405 A1

### Non-Patent Document

Non-Patent Document 1: Lamb, R. A. and Kolakofsky, D., Fundamental Virology, 4th edition (Lippincott Williams & Wilkins), pp689 - 724.
Non-Patent Document 2: Nishimura, K., et al., J. Biol. Chem., 286, 4760-4771, 2011
Non-Patent Document 3: Masaki, I., et al., Circulation Research, 90, 966-973, 2002
Non-Patent Document 4: Harris, H. Nature, 206, 583, 1965
Non-Patent Document 5: Nakanishi, M. and Otsu, M. Current Gene Therapy, 12, 410-416, 2012
Non-Patent Document 6: Gething, M.J., et al., Proc. Natl. Acad. Sci. USA, 75, 2737-2740, 1978
Non-Patent Document 7: Kershaw, M.H., et al., Nature Reviews Cancer, 13, 525, 2013
Non-Patent Document 8: Cole, S.P., et al., Mol. Cell. Biochem., 62, 109-120, 1984
Non-Patent Document 9: Allan, S.E., et al., Molecular Therapy, 16, 194-202, 2008
Non-Patent Document 10: Watabe, A., et al., Biochim. Biophys. Acta, 1416, 339-348, 1999
Non-Patent Document 11: Eguchi, a., et al., J. Biol. Chem., 275, 17549-17555, 2000
Non-Patent Document 12: Naldini, L., et al., Proc. Natl. Acad. Sci. USA, 93, 11382-11388, 1996
Non-Patent Document 13: Funke, S., et al., Molecular Therapy, 16, 1427-1436, 2008
Non-Patent Document 14: Stone, R., et al., PLOS One, 8(4), e61281, 2013
Non-Patent Document 15: Gosselin-Grenet, A-S., et al., Virology, 405, 439-447, 2010

### Summary of the Invention

### Problems to be Solved by the Invention

Therefore, the problem to be solved by the present invention is to construct a modified envelope protein effective for pseudo-typing. The goal is to realize pseudo-typing by an envelope protein derived from a morbillivirus belonging to the Family *Paramyxoviridae* in a negative-sense single-stranded RNA virus vector comprising an RNA-dependent RNA polymerase derived from the Genus *Respirovirus* belonging to the Family *Paramyxoviridae.* This aims to realize efficient gene transfer and expression into lymphocytes such as B cells, CD4 positive T cells, and CD8 positive T cells contained in peripheral blood, and immortalized cells derived from these cells.

### Means for Solving the Problems

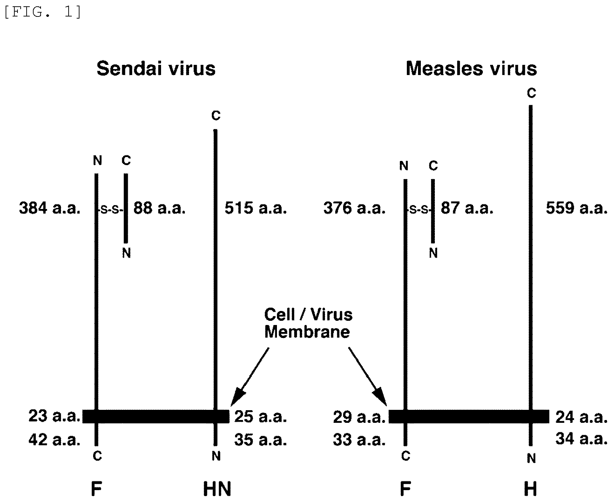

When the structures and functions of an F protein (555 amino acid residues) of a Sendai virus Z strain (GenBank #M30202.1) and an F protein (553 amino acid residues) of a Measles virus Edmonston strain (GenBank #K01711.1) are compared, both have a common feature that the C-terminal side is located inside the envelope (virus particle inner side; also referred to as a cytoplasm side), and both become an active form that is cleaved by a protease to induce membrane fusion (FIG. 1). However, the identity of the primary structure is only 30.0%.

When the structures and functions of the HN protein (576 amino acid residues) of the Sendai virus Z strain (GenBank # M30202.1) and the H protein (617 amino acid residues) of the Measles virus Edmonston strain (GenBank # K01711.1) are compared, both have a common feature that the N-terminal side is located on the inner side of the envelope (the inner side of the virus particle; on the cytoplasmic side) and is responsible for binding activity to the cell surface (FIG. 1). The HN protein of the Sendai virus binds to sialic acid (Non-Patent Document 1), and the H protein of the MeV Edmonston strain binds to CD46 and SLAM on the cell surface (Tatsuo, H., et al., Nature, 406, 893-897, 2000). The identity of their primary structures is as low as 19.8%.

In addition to the F protein and the HN protein which are envelope membrane proteins, the M protein present inside the envelope is required for the formation of the Sendai virus particles (Kondo, T., et al., J. Biol. Chem., 268, 21924-21930, 1993). Genes encoding these three types of proteins are not present in the genome of the SeVdp vector or the stealth RNA vector, and vector particles are produced by supplementing proteins with the expression of genes transferred from the outside in the form of plasmid. In this case, it has been reported that vector particles of SeVdp cannot be produced in the absence of plasmid expressing the M protein (Non-Patent Document 2).

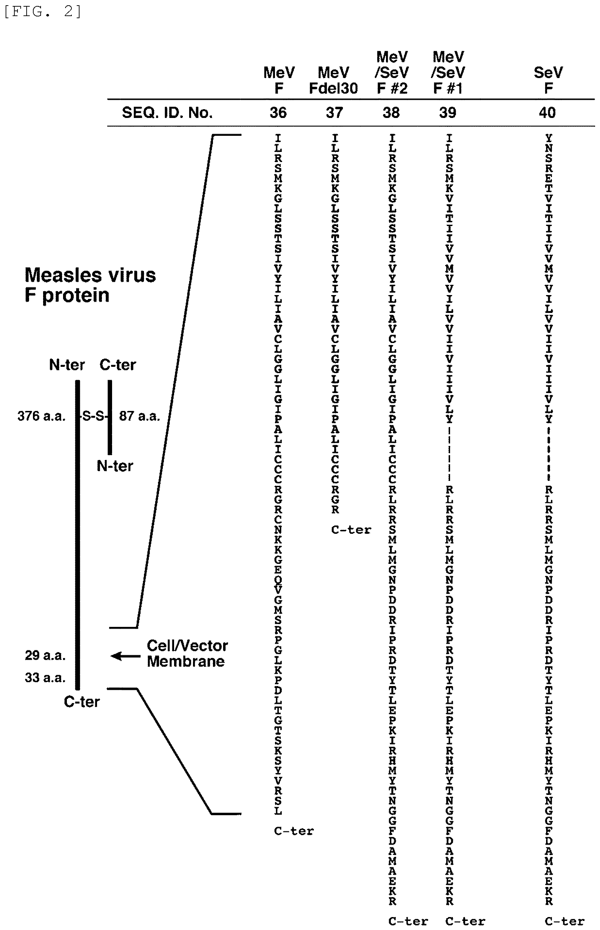

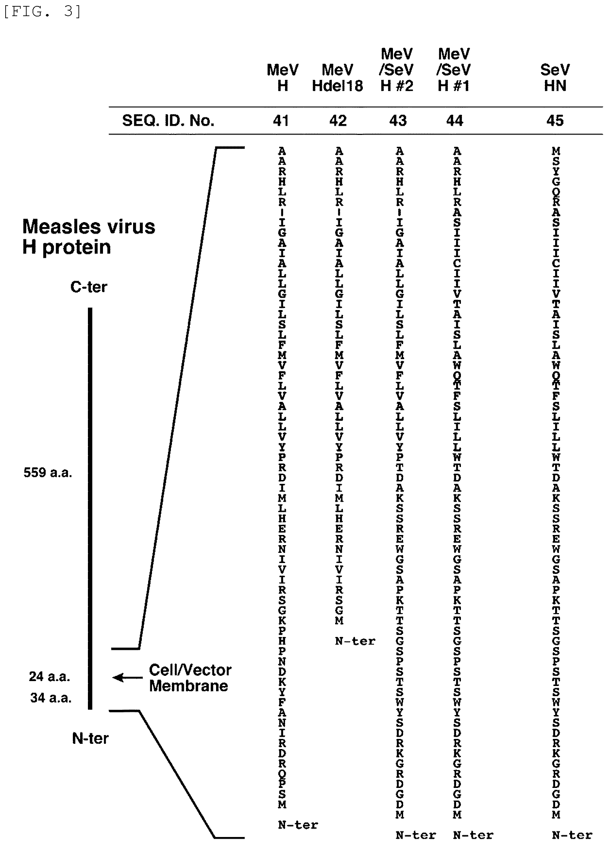

An M protein performs particle formation by binding both an NP protein covering virus genomic RNA and an envelope glycoprotein. The envelope glycoprotein interacts with the M protein via a domain on its cytoplasmic side (inner side of the virus particle). However, the identity between the cytoplasmic side domain of the F protein of the Sendai virus and the cytoplasmic side domain of the MeV F protein is only 14.3% (FIG. 2). Similarly, the identity between the cytoplasmic domain of the HN protein of the Sendai virus and the cytoplasmic domain of the MeV H protein is only 18.9% (FIG. 3). For this reason, the MeV envelope glycoproteins (F and H) are less likely to bind to the M protein of the Sendai virus.

Therefore, it is considered that by substituting the cytosolic domain of the MeV envelope glycoprotein with the cytosolic domain of the envelope glycoprotein of the Sendai virus, the modified MeV envelope glycoprotein and the M protein of the Sendai virus can bind to each other for pseudo-typing. Meanwhile, there is a result of a previous study that even when the cytosolic domain of the MeV H protein is substituted with the cytosolic domain of the HN protein of the Sendai virus, uptake into the Sendai virus particles does not occur (Non-Patent Document 15), and it is expected that the above problem cannot be solved only by simply replacing the cytosolic domain of the envelope glycoprotein.

Therefore, the present inventors variously combined the MeV F protein (FIG. 2) in which the cytoplasmic domain and the cell transmembrane domain were modified with the MeV H protein (FIG. 3) in which the cytoplasmic domain and the cell transmembrane domain were modified with each other, expressed in a cell having the genome of a stealth RNA vector together with the M protein of the Sendai virus, and examined whether a pseudotyped stealth RNA vector was produced. As a result, it was found that pseudotyped stealth RNA vectors capable of infecting Vero cells of 3 x 10⁵ Cell Infectious Units (CIU)/mL or more, which is a practical standard, can be produced by 5 kinds of combinations (FIG. 7).

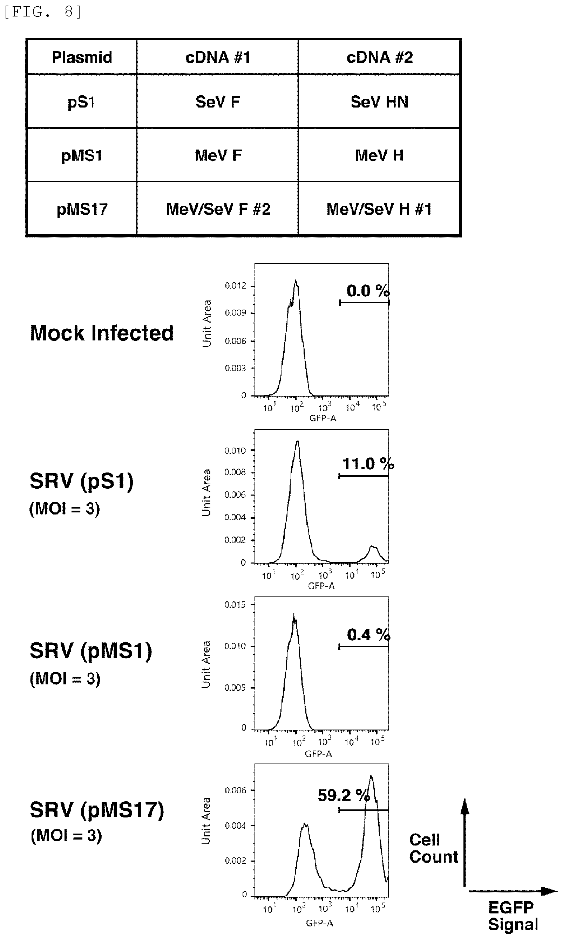

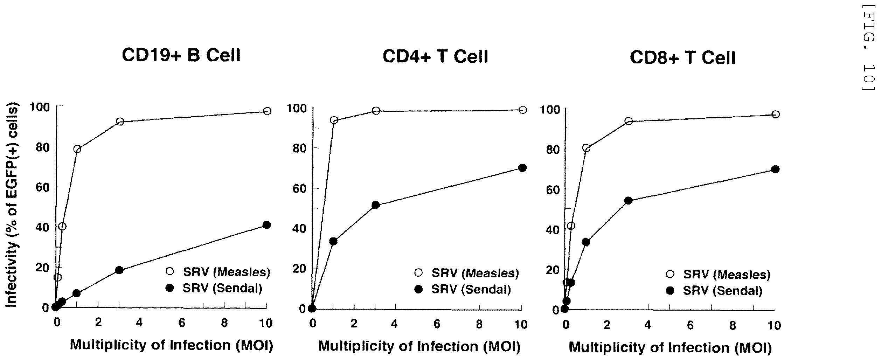

Furthermore, the abilities of these five pseudotyped stealth RNA vectors to be transferred into cells derived from blood were compared, and it was found that only a pseudotyped stealth RNA vector produced by combining a specific modified F protein (MeV/SeV F#2) and a modified H protein (MeV/SeV H#1) can be transferred with high efficiency (Table 1, FIG. 8). This pseudotyped stealth RNA vector was capable of transferring genes into 90% or more of human peripheral blood-derived B cells, CD4 positive T cells, and CD8 positive T cells with the gene at Multiplicity of infection (MOI) = 3 (FIG. 10).

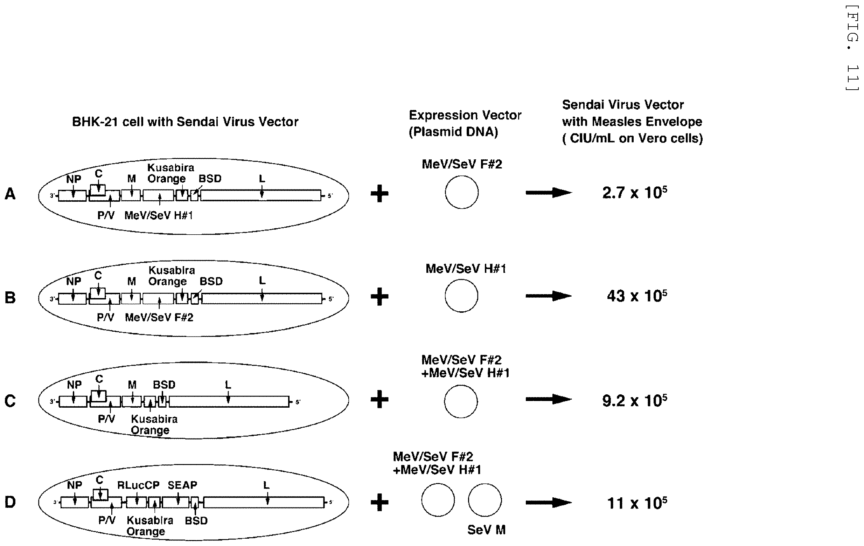

It was found that not only stealth RNA vectors but also Sendai virus vectors can be pseudotyped by using MeV/SeV F#2 and MeV/SeV H#1 in combination (FIG. 11), and the problem could be completely solved.

Preferred embodiments of the present invention are illustrated below.
[1] A chimeric F protein of a paramyxovirus, which is any of the following (1) to (8):
   (1) a polypeptide composed of an amino acid sequence encoded by a base sequence of SEQ ID NO: 3,
   (2) a polypeptide comprising an amino acid sequence encoded by a base sequence of SEQ ID NO: 3,
   (3) a polypeptide composed of an amino acid sequence encoded by a base sequence of SEQ ID NO: 24,
   (4) a polypeptide comprising an amino acid sequence encoded by a base sequence of SEQ ID NO: 24,
   (5) a polypeptide composed of an amino acid sequence of SEQ ID NO: 33,
   (6) a polypeptide comprising an amino acid sequence of SEQ ID NO: 33,
   (7) a polypeptide composed of an amino acid sequence of SEQ ID NO: 34, and
   (8) a polypeptide comprising an amino acid sequence of SEQ ID NO: 34.
[2] A combination of proteins comprising the chimeric F protein of a paramyxovirus in the above [1] and an H/HN chimeric protein of a paramyxovirus which is any one of the following (1) to (8):
   (1) a polypeptide composed of an amino acid sequence encoded by a base sequence of SEQ ID NO: 9,
   (2) a polypeptide comprising an amino acid sequence encoded by a base sequence of SEQ ID NO: 9,
   (3) a polypeptide composed of an amino acid sequence of SEQ ID NO: 35,
   (4) a polypeptide comprising an amino acid sequence of SEQ ID NO: 35,
   (5) a polypeptide composed of an amino acid sequence encoded by a base sequence of SEQ ID NO: 50,
   (6) a polypeptide comprising an amino acid sequence encoded by a base sequence of SEQ ID NO: 50,
   (7) a polypeptide composed of an amino acid sequence of SEQ ID NO: 51, and
   (8) a polypeptide comprising an amino acid sequence of SEQ ID NO: 51.
[3] A vector capable of expressing a chimeric F protein of a paramyxovirus, the vector being any one of the following (1) to (4):
   (1) a vector comprising a polynucleotide having a base sequence of SEQ ID NO: 3,
   (2) a vector comprising a polynucleotide having a base sequence of SEQ ID NO: 24,
   (3) a vector comprising a polynucleotide encoding a polypeptide having an amino acid sequence of SEQ ID NO: 33, and
   (4) a vector comprising a polynucleotide encoding a polypeptide having an amino acid sequence of SEQ ID NO: 34.
[4] The vector according to the above [3], further comprising any one of the following polynucleotides (1) to (4):
   (1) a polynucleotide having a base sequence of SEQ ID NO: 9,
   (2) a polynucleotide encoding a polypeptide having an amino acid sequence of SEQ ID NO: 35,
   (3) a polynucleotide having a base sequence of SEQ ID NO: 50, and
   (4) a polynucleotide encoding a polypeptide having an amino acid sequence of SEQ ID NO: 51.
[5] The vector according to the above [3], wherein the vector is a plasmid vector.
[6] The vector according to the above [4], wherein the vector is a plasmid vector.
[7] A combination of vectors comprising a vector capable of expressing a chimeric F protein of a paramyxovirus and a vector capable of expressing a chimeric H/HN protein of a paramyxovirus, including the vector of the above [3] and any of the following vectors (1) to (4):
   (1) a vector comprising a polynucleotide having a base sequence of SEQ ID NO: 9,
   (2) a vector comprising a polynucleotide encoding a polypeptide having an amino acid sequence of SEQ ID NO: 35,
   (3) a vector comprising a polynucleotide having a base sequence of SEQ ID NO: 50; and
   (4) a vector comprising a polynucleotide encoding a polypeptide having an amino acid sequence of SEQ ID NO: 51.
[8] The combination of the vectors of the above [7], wherein the combination is a combination of plasmid vectors.
[9] A host cell transformed with the vector according to any one of the above [3] to [6] or the combination of the vectors according to the above [7] or [8].
[10] The transformed host cell according to the above [9], wherein the host cell is a eukaryotic cell.
[11] A pseudotyped virus particle having a negative-sense single-stranded RNA genome, comprising the chimeric F protein according to the above [1] or the combination of proteins according to the above [2] as an envelope protein.
[12] The virus particle according to the above [11], wherein the negative-sense single-stranded RNA genome includes a cRNA sequence encoding exogenous protein(s).
[13] A method for transferring a gene into a lymphocyte derived from human peripheral blood, the method comprising the process of contacting the lymphocyte derived from the human peripheral blood with the virus particle according to the above [12].
[14] The method according to the above [13], wherein the lymphocyte is a lymphocyte selected from the group consisting of a B cell, a CD4 positive T cell, and a CD8 positive T cell.
[15] The method according to the above [13], wherein the gene transfer is delivery of reprogramming genes, and the method is capable of establishing induced pluripotent stem cells (iPS cells).
[16] The method according to the above [14], wherein the gene transfer is delivery of reprogramming genes, and the method is capable of establishing induced pluripotent stem cells (iPS cells).
[17] The method according to the above [13], wherein the lymphocyte is an immortalized lymphocyte.
[18] The method according to the above [14], wherein the lymphocyte is an immortalized lymphocyte.
[19] The virus particle according to the above [11], wherein the negative-sense single-stranded RNA genome is genomic RNA of a paramyxoviridae virus other than a morbillivirus, or a variant thereof.
[20] The virus particle according to the above [19], wherein the variant is a variant in which one or more of a virus endogenous gene encoding an envelope protein and a virus endogenous gene encoding an M protein are functionally deleted.
[21] The virus particle according to the above [20], wherein the virus endogenous gene encoding the envelope protein is an F gene and/or an H gene, an HN gene, or a G gene.

### Effects of the Invention

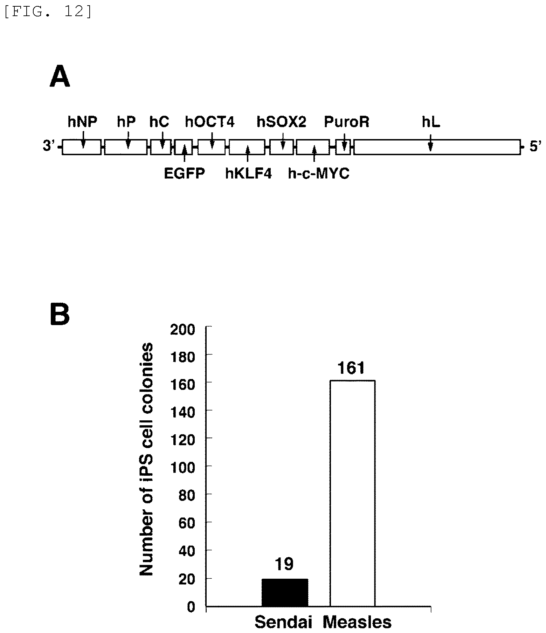

According to the present invention, host cell specificity of a Sendai virus vector or a stealth RNA vector is improved, and gene transfer into blood cells including B cells, CD4 positive T cells and CD8 positive T cells, which have been inefficient so far, is facilitated. Therefore, it has a great impact on an industrial field where application of a negative-sense single-stranded RNA virus vector has been expected, such as a change in properties of blood cells including initialization and immortalization, and expression of a chimeric antigen receptor (CAR) on T cells. In particular, it is expected that research and development using B cells for which there has been almost no application example so far will be accelerated. For example, in the establishment of induced pluripotent stem cells derived from B cells, which has been difficult so far, the efficiency could be raised nearly 10 times (Example 16, FIG. 12).

### Brief Description of Drawings

FIG. 1 is a diagram comparing the structures of Envelope proteins of a Sendai virus and a Measles virus.
FIG. 2 is a diagram showing the primary structure of an MeV/SeV chimeric F protein.
FIG. 3 is a diagram showing the primary structure of an MeV/SeV chimeric H (H/HN) protein.

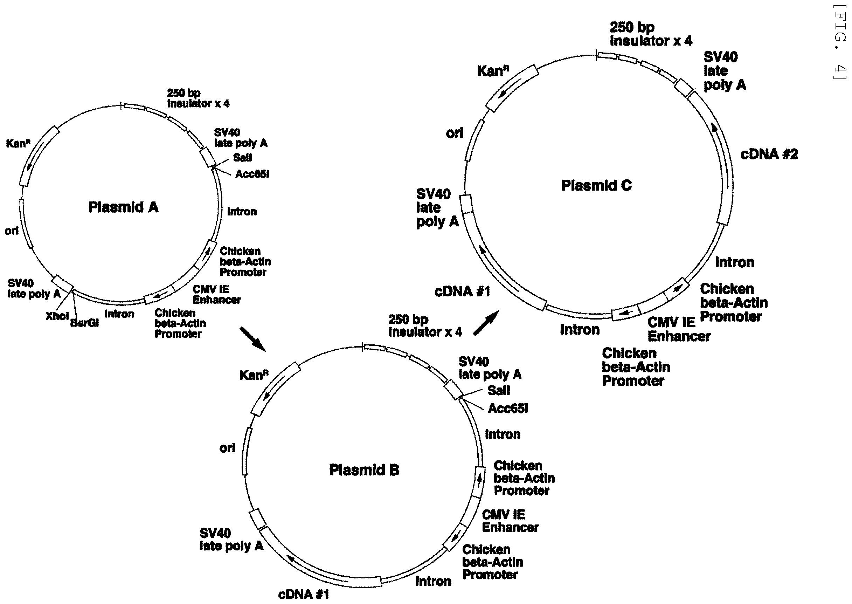

FIG. 4 is a diagram showing the construction of plasmid C that simultaneously expresses an F protein and an H protein.

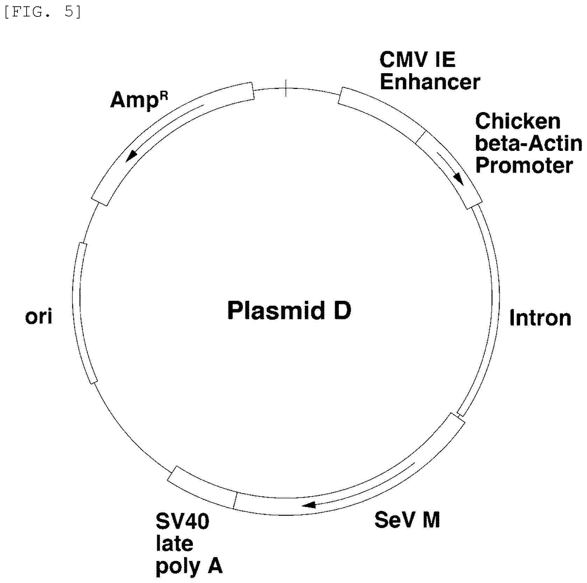

FIG. 5 is a diagram showing the structure of plasmid expressing the M protein of a Sendai virus.

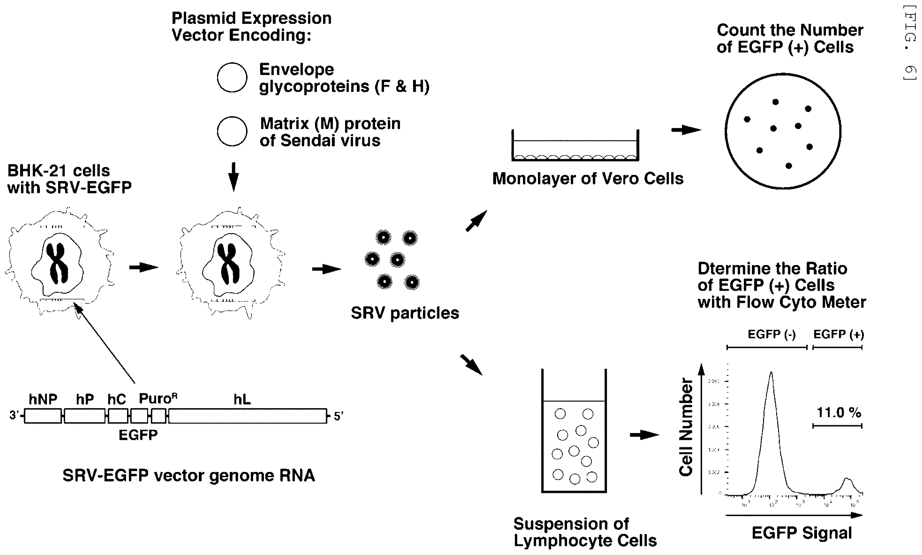

FIG. 6 is a diagram showing a method for measuring the gene transfer activity of a stealth RNA vector (SRV) using monolayer cultured cells and a method for measuring the gene transfer activity using suspension cells.

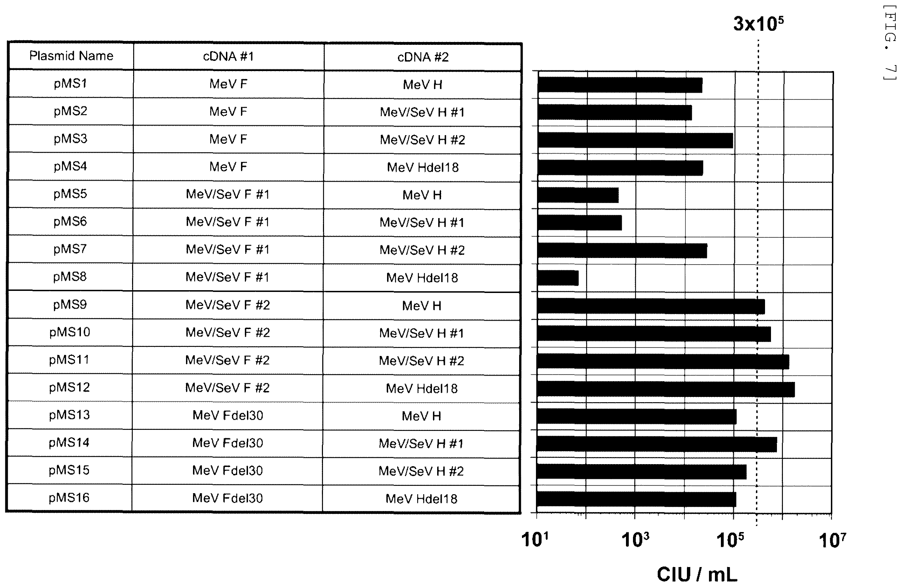

FIG. 7 is a diagram showing the comparison of the production amounts of stealth RNA vectors pseudotyped with MeV/SeV chimeric proteins (quantification of the number of infected cells using Vero cells).
FIG. 8 is a diagram showing gene transfer efficiency into Daudi cells by stealth RNA vectors pseudotyped with MeV/SeV chimeric proteins (quantification by flow cytometer with EGFP expression as an index).

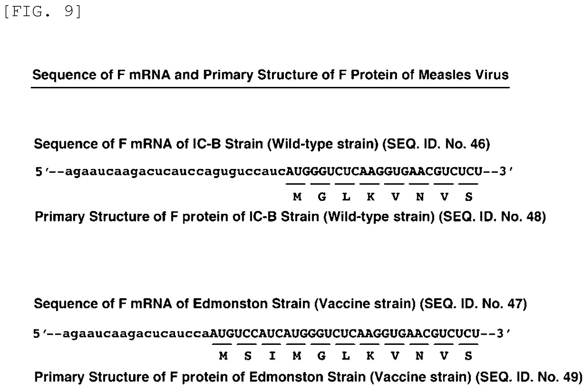

FIG. 9 is a view comparing the structures near the N-terminus of F protein of a Measles virus wild-type strain and a vaccine strain.
FIG. 10 shows the comparison of gene transfer efficiencies into primary cultured peripheral blood lymphocytes by stealth RNA vectors pseudotyped with MeV/SeV chimeric proteins (quantification by flow cytometer with EGFP expression as an index).
FIG. 11 is a diagram showing the comparison of the production amounts of Sendai virus vectors having MeV/SeV chimeric proteins (quantification of the number of infected cells using Vero cells).
FIG. 12 is a diagram showing the efficiency of induction of iPS cells from B lymphocytes by stealth RNA vectors pseudotyped with MeV/SeV chimeric proteins and carrying genes encoding 4 reprogramming factors.

### Description of Embodiments

One embodiment of the present invention is an RNA virus vector having a gene expression system having negative-sense single-stranded RNA as a genome therein, and having a chimeric F protein (chimerized with an F protein of all Paramyxoviridae viruses except a morbillivirus, such as Genus *Respirovirus*) of a morbillivirus and a chimeric H protein (chimerized with an H protein of all Paramyxoviridae viruses except a morbillivirus, such as Genus *Respirovirus*) of a morbillivirus in the outer membrane, thereby enhancing the directivity to blood cells. Preferable examples of the chimeric F protein include the polypeptides of (1) to (8) in the above [1]. Preferable examples of the chimeric H protein include the polypeptides of (1) to (8) in the above [2]. More preferred examples of the chimeric H protein are the polypeptides of (1) to (4) in the above [2]. In a preferred embodiment for enhancing the host cell specificity of the RNA virus vector to blood cells, as described in the above [2], a chimeric F protein which is the polypeptide of any one of (1) to (8) in the above [1] and a chimeric H protein which is the polypeptide of any one of (1) to (8) in the above [2] (more preferably, any one of (1) to (4) in the above [2]) are used in combination. The nucleic acid encoding the chimeric F protein or chimeric H protein may be DNA or RNA. The DNA or RNA is preferably used by being incorporated into a plasmid vector or an RNA virus vector (more specifically, the negative-sense single-stranded RNA genome contained in the RNA virus vector) in a manner capable of expressing a chimeric F protein or a chimeric H protein. Specific examples of preferred vectors include the vectors described in [3] to [8]. When the nucleic acid encoding the chimeric F protein and/or the chimeric H protein is not contained in the negative-sense single-stranded RNA genome, the negative-sense single-stranded RNA genome (or plasmid DNA capable of transcribing the negative-sense single-stranded RNA genome) and the expression vector described in any one of [3] to [8] can be used in combination in constructing the RNA virus vector (virus particle). As described in the above [11] and [12], the virus particle of the present invention is the pseudotyped virus particle. Since the pseudotyped virus particle of the present invention has enhanced host cell specificity to blood cells, particularly to lymphocytes and cells derived from lymphocytes, gene transfer into lymphocytes can be efficiently performed through the process of bringing lymphocytes (in particular, lymphocytes derived from human peripheral blood selected from the group consisting of B cells, CD4 positive T cells, and CD8 positive T cells, and immortalized lymphocytes) into contact with the virus particle of the present invention as described in [13] to [18]. Here, when reprogramming genes are transferred using a lymphocyte selected from the group consisting of B cells, CD4 positive T cells, and CD8 positive T cells as a host, induced pluripotent stem cells (iPS cells) can be established with much higher efficiency than in the prior art.

The negative-sense single-stranded RNA may be genomic RNA of all paramyxoviridae viruses other than a morbillivirus, or a variant thereof. Examples of the paramyxoviridae virus other than the morbillivirus include a Sendai virus, a human parainfluenza virus, and a bovine parainfluenza virus of the Genus *Respirovirus.* Other examples of the paramyxoviridae virus other than the morbillivirus include a mumps virus, a Newcastle disease virus, an avian paramyxovirus, a hendra virus, and a Nipah virus. A representative example of a morbillivirus is, but is not limited to, a Measles virus. Here, the variants include all variants such as a variant in which one or more endogenous genes of paramyxoviridae virus selected from genes encoding the envelope protein (F and H, HN or G) and the M protein, are deleted, and a variant in which a gene cassette for expressing one or more exogenous genes such as reprogramming genes are introduced. As described above, the RNA vector of the present invention may have, as a genomic RNA, a negative-sense single-stranded RNA which is a genomic RNA of all paramyxoviridae viruses except for the morbillivirus or a variant thereof. However, in consideration of industrial applications and pathogenicity to humans, a vector having a negative-sense single-stranded RNA as a genome, which is structurally optimized as described in Patent Document 4, or a vector having a genomic RNA of a Sendai virus is optimal.

In Examples, a chimeric protein made of an H protein of a vaccine strain of a Measles virus (Edmonston strain), which is a representative morbillivirus, and an HN protein of a Sendai virus was mainly used, but the embodiment for carrying out the invention is not limited thereto.

For example, as in Examples described later, it is possible to use a chimeric protein comprising, as materials, an H protein (GenBank #NC_001498.1) of a wild epidemic strain of a Measles virus (wild-type strain; IC-B strain, Takeuchi, K., et al., Virus Genes, 20, 253-257, 2000) using SLAM as a receptor and an HN protein of a Sendai virus. In addition, a combination of a chimeric protein comprising, as materials, an H protein of a canine distemper virus (GenBank #AF014953.1) and an HN protein of a Sendai virus and a chimeric protein comprising, as materials, an F protein of a canine distemper virus (GenBank #AF014953.1) and an F protein of a Sendai virus is also very suitable for gene transfer into blood cells.

A Measles virus is a representative morbillivirus that can infect only humans and monkeys, but the Genus *Morbillivirus* includes a very large number of animal species-specific viruses. Examples thereof include canine distemper virus, cowpox virus, feline morbillivirus, seal distemper virus, dolphin morbillivirus, and peste-des-petits-ruminants virus (Non-Patent Document 1). Also in these morbilliviruses, the basic structures and functions of the F protein and the H protein are the same as those of the Measles virus, and if chimeric proteins are constructed using envelope proteins derived from these other species of morbilliviruses, RNA virus vectors for gene transfer specialized for animal species can be produced.

Production of vector particles having a chimeric F protein and a chimeric H protein having a region derived from a morbillivirus in the outer membrane, including expression of the chimeric F protein and the chimeric H protein, is possible by supplying all of the F, H, and M proteins from the outside by an expression vector using plasmid DNA, as shown in Example 5, but is not limited thereto. Even when one or two of the genes encoding these proteins are carried on a single-stranded RNA genome, pseudotyped virus vectors of the same structure can be produced (Example 15).

All of the chimeric F protein, the chimeric H protein, and a combination of these chimeric proteins according to the present invention, one or more vectors capable of expressing the chimeric protein, a host cell transformed with the one or more vectors, production of a recombinant chimeric protein using the host cell, RNA virus particles comprising the one or more chimeric proteins as an envelope protein, an unmodified or modified single-stranded RNA virus genome contained in the virus particles, a method for gene transfer into a lymphocyte derived from human peripheral blood by contacting the virus particles with a lymphocyte derived from human peripheral blood, and iPS cells established (initialized) by gene transfer of reprogramming genes by the method can be appropriately carried out by those skilled in the art based on examples of specific and detailed embodiments in the following Examples.

In practice, those skilled in the art can appropriately refer to and use general molecular biological techniques and general knowledge and techniques in the technical field described in the prior art such as Patent Document 4.

Examples of the present invention are disclosed below. However, the present invention is not limited to Examples.

### Example 1

### Production of cDNA encoding chimeric F protein of Measles virus

An F protein of Measles virus Edmonston strain is synthesized in an endoplasmic reticulum as a precursor of 553 amino acid residues, and undergoes cleavage in the process of being transported to a cell membrane via a golgi apparatus to become active (FIG. 1). The C-terminal 33 amino acid residues of the F protein are present inside the virus outer membrane, and the immediately N-terminal 29 amino acid residues are hydrophobic regions present in the lipid bilayer membrane (Morrison, T. and Portner, A., In "The Pramyxoviruses", Kingsbury, D. W., ed., pp347-382). Therefore, in order to express various chimeric F proteins, the following cDNA was produced. Each cDNA was codon-optimized by OptimumGen Gene Design System (US Patent 8326547), and then synthesized by adding GGTACCACC on the 5' side and CTCGAG on the 3' side.
1. MeV F: cDNA encoding F protein of Measles virus Edmonston strain (SEQ ID NO: 1)
2. MeV Fdel30: cDNA encoding F protein of a modified Measles virus Edmonston strain with deletion of 30 amino acid residues on the C-terminal side (SEQ ID NO: 2)
3. MeV/SeV F#2: cDNA encoding F protein of a modified Measles virus Edmonston strain (SEQ ID NO: 33), in which the C-terminal 33 amino acid residues were substituted with the C-terminal 42 amino acid residues of F protein of Sendai virus Z strain (SEQ ID NO: 3)
4. MeV/SeV F#1: cDNA encoding F protein of a modified Measles virus Edmonston strain, in which the C-terminal 62 amino acid residues were substituted with the C-terminal 65 amino acid residues of F protein of Sendai virus Z strain (SEQ ID NO: 4)
5. SeV F: cDNA encoding an F protein of a modified Sendai virus Z strain (Taira, H., et al., Arch. Virol., 140, 187-194, 1995) in which four amino acid residues at the cleavage site were substituted to increase the sensitivity to Furin (SEQ ID NO: 5)

### Example 2

### Production of cDNA encoding chimeric H protein of Measles virus

An H protein of Measles virus Edmonston strain is composed of 559 amino acid residues (FIG. 1). The N-terminal 34 amino acid residues of the H protein are present inside a virus outer membrane, and the immediately C-terminal 24 amino acid residues are hydrophobic regions present in a lipid bilayer membrane (Morrison, T. and Portner, A., In "The Pramyxoviruses", Kingsbury, D. W., ed., pp347-382). Therefore, in order to express various chimeric H proteins, the following cDNA was produced. Each cDNA was codon-optimized by OptimumGen Gene Design System (US 8326547), and then synthesized by adding GGTACCACC on the 5' side and CTCGAG on the 3' side.
1. MeV H: cDNA encoding H protein of Measles virus Edmonston strain (SEQ ID NO: 6)
2. MeV Hdel18: cDNA encoding an H protein of Measles virus modified Edmonston strain with deletion of 18 amino acid residues at the N-terminal side (SEQ ID NO: 7)
3. MeV/SeV H#2: cDNA encoding an H protein of a modified Measles virus Edmonston strain, in which the N-terminal 34 amino acid residues were substituted with the N-terminal 35 amino acid residues of an HN protein of Sendai virus Z strain (SEQ ID NO: 8)
4. MeV/SeV H#1: cDNA encoding an H protein of a modified Measles virus Edmonston strain (SEQ ID NO: 35), in which the N-terminal 58 amino acid residues were substituted with the N-terminal 60 amino acid residues of an HN protein of Sendai virus Z strain (SEQ ID NO: 9)
5. SeV HN: cDNA encoding an HN protein of Sendai virus Z strain (SEQ ID NO: 10)

### Example 3

### Production of plasmid vector expressing chimeric F protein and chimeric H protein of Measles virus

A pGEM5-Zf (+) vector (Promega Corporation, GenBank #X65308) was cleaved with restriction enzymes ApaI and NcoI, treated with S1 Nuclease, and then the cleavage site was bound with T4 DNA ligase to produce Plasmid_#1. Next, using plasmid pact-c-myb (Nishina, Y., et al., Nucl.Acid Res., 17, 107-117, 1989) as a template, a DNA fragment comprising a chicken beta actin promoter was amplified by a polymerase chain reaction (PCR) method using Primer_#1 (SEQ ID NO: 11), Primer _#2 (SEQ ID NO: 12), and PrimeSTAR DNA polymerase (TAKARA Bio Inc.). The Plasmid _#1 was cleaved with restriction enzymes NotI and NdeI, and this amplified DNA was inserted to produce the Plasmid _#2. Next, the Plasmid _#2 was cleaved with restriction enzymes SalI and XhoI, and synthetic DNA (SEQ ID NO: 13) comprising an early gene enhancer of cytomegalovirus (CMV IE enhancer) was inserted to produce Plasmid_#3. Next, the Plasmid _#3 was cleaved with restriction enzymes BsrGI and NsiI, and synthetic DNA (SEQ ID NO: 14) comprising a late gene transcription termination signal of SV40 was inserted to prepare Plasmid_#4. By site-directed mutagenesis using Primer _#3 (SEQ ID NO: 15), Primer _#4 (SEQ ID NO: 16), and QuikChange Lightning Multi Site-Directed Mutagenesis Kit (Agilent Technologies, Inc.), the restriction enzyme BsrGI cleavage site (TGTACA) in the Plasmid_#4 was replaced with GGTACC, and the restriction enzyme XhoI cleavage site (CTCGAG) was replaced with GTCGAC to produce Plasmid _#5.

Next, using this Plasmid_#5 as a template, a DNA fragment comprising a CMV IE enhancer · beta actin promoter · intron · SV40 late gene transcription termination signal was amplified by a PCR method using Primer_#5 (SEQ ID NO: 17) and Primer _#6 (SEQ ID NO: 18), and the Plasmid _#5 was inserted into a site cleaved with restriction enzymes NotI and SalI to produce Plasmid_#6.

Next, the Plasmid _#6 was cleaved with the restriction enzyme NotI, and a synthetic DNA fragment (SEQ ID NO: 19) comprising 4 copies of a 250bp insulator sequence (Recillas-Targa, F., et al., Proc. Natl. Acad. Sci. USA, 99, 6883-6888, 2002) derived from a chicken globin gene in the same direction was inserted to produce Plasmid_#7. Finally, the Plasmid _#7 was cleaved with a restriction enzyme BspHI, and synthetic DNA comprising a kanamycin resistance gene (SEQ ID NO: 20) was inserted to complete Plasmid A (SEQ ID NO: 21) (FIG. 4). Using this plasmid, two cDNAs can be expressed simultaneously and potently.

Plasmid A was cleaved with restriction enzymes BsrGI and XhoI, and a DNA fragment obtained by cleaving the cDNA encoding a chimeric F protein described in Example 1 with restriction enzymes Acc65I and XhoI was inserted to prepare Plasmid B (FIG. 4; In the figure, cDNA #1 represents a cDNA insert encoding a chimeric F protein). Furthermore, Plasmid B was cleaved with restriction enzymes Acc65I and SalI, and a DNA fragment obtained by cleaving the cDNA encoding a chimeric H protein described in Example 2 with restriction enzymes Acc65I and XhoI was inserted to prepare Plasmid C (FIG. 4; In the figure, cDNA #2 represents a cDNA insert encoding a chimeric H protein). All of pMS1 to pMS16 (FIG. 7), pMS17 (Table 2), and pS1 (FIG. 9) represent plasmids corresponding to the Plasmid C, and the types of cDNA carried on the plasmids are shown in FIG. 7, Table 2, and FIG. 9. Each plasmid DNA was prepared in a large amount using E. coli NEB Stable strain (New England BioLab), purified by a cesium chloride density gradient method and a phenol extraction-chloroform extraction-ethanol precipitation method, dissolved in a TE buffer (10 mM Tris-HCl, 1 mM EDTA, pH = 8), and stored at 4°C.

### Example 4

### Production of plasmid vector expressing M protein of Sendai virus

A cDNA encoding the M protein of Sendai virus Clone 151 strain (GenBank #AB275416) was codon-optimized by OptimumGen Gene Design System (US 8326547), and then synthesized by adding GGTACCACC on the 5' side and CTCGAG on the 3' side (SEQ ID NO: 22).

The Plasmid_#4 prepared in Example 3 was cleaved by restriction enzymes BsrGI and XhoI, and a DNA fragment comprising M protein cDNA cleaved by restriction enzymes Acc65I and XhoI was inserted to prepare Plasmid D (FIG. 5). The Plasmid D was prepared in a large amount using E. coli NEB-alpha strain (New England BioLabs, Inc.), purified by a cesium chloride density gradient method and a phenol extraction-chloroform extraction-ethanol precipitation method, dissolved in a TE buffer (10 mM Tris-HCl, 1 mM EDTA, pH=8), and stored at 4°C.

### Example 5

### Production of stealth RNA vector pseudotyped with chimeric F protein of Measles virus and chimeric H protein of Measles virus

A template cDNA of a stealth RNA vector (SRV-EGFP) carrying Enhanced Green Fluorescent Protein (EGFP) (Cormack, B.P., et al., Gene, 173, 33-38, 1996) and a puromycin resistance gene (Lacalle, R. A., et al., Gene, 79, 375-380, 1989) was prepared according to Example 5 of Patent Document 4. Specifically, a synthetic DNA fragment (SEQ ID NO: 23) comprising an EGFP gene and a puromycin resistance gene was used instead of the DNA fragment comprising 10 genes, and this DNA was cleaved with XmaI and NotI to prepare template cDNA. Next, SRV-EGFP was produced using this template cDNA according to Example 6 of Patent Document, and genes were transferred into BHK-21 cells derived from hamster (Macpherson, I. and Stoker, M., Virology, 16, 147-151, 1962). BHK-21 cells with SRV-EGFP genome retained in cytoplasm (BHK-21/SRV-EGFP cells) were cultured in Dulbecco's modified Eagle's medium (Dulbecco's modified Eagle's medium, DMEM medium) (Merck, #D5796) containing 2 µg/mL of puromycin (Calbiochem) and 10% fetal bovine serum (Global Life Sciences Solutions USA LLC), and all cells were confirmed to be EGFP positive by a fluorescence microscope (Carl Zeiss, AxioVert A1 type).

BHK-21/SRV-EGFP cells were seeded on a 12 well plate at 1 x 10⁵ cells/well/800 µL, and cultured for 1 day. The next day, 1.6 µg of Plasmid (pMS1 to pMS17) expressing a chimeric F protein and a chimeric H protein, 0.8 µg of Plasmid D, 1.6 µL of Lipofectamine PLUS reagent (Thermo Fisher Scientific Inc.), and 4 µL of Lipofectamine LTX (Thermo Fisher Scientific Inc.) were mixed in 100 µL of an Opti-MEM medium (Thermo Fisher Scientific Inc.), and the mixture was reacted at room temperature for 10 minutes. The medium containing this DNA was added to BHK-21/SRV-EGFP cells, and a DMEM medium containing 0.7 mL of 10% fetal bovine serum was added thereto, and the cells were cultured for another 1 day (FIG. 6).

Next, the medium was replaced with the DMEM medium containing fresh 10% fetal bovine serum to reduce the culture temperature to 32°C, and the cells were cultured for another 3 days to recover a culture supernatant containing a pseudotyped vector. The collected supernatant was filtered through a 0.45 µm filter and then stored at 4°C. When it was necessary to store the supernatant for 2 days or more, the supernatant was cryopreserved at -80°C.

### Example 6

### Measurement of infectivity of pseudotyped stealth RNA vectors using Vero cells

Vero cells (Shimizu, B., et al., Proc. Soc. Exp. Biol. Med., 125, 119-123, 1967), as a cell strain derived from African green monkey kidney, were cultured in Eagle's minimum essential medium (Eagle's minimum essential medium, MEM medium) containing 10% fetal bovine serum (Merck, #M4655), and seeded in 48 well plates at 2.5 x 10⁴ cells/well/200 µL. After 24 hours, the medium was replaced with 150 µL of a solution obtained by diluting the pseudotyped vector produced in Example 5 with MEM medium. After culturing for another 48 hours, the number of EGFP positive cells was counted under a fluorescence microscope (FIG. 6). The number of vector particles capable of transferring the EGFP gene into a single Vero cell and expressing the EGFP gene was defined as 1 Cell Infectious Unit (CIU), and the concentration of the vector was quantified by CIU/mL.

### Example 7

### Measurement of infectivity of pseudotyped stealth RNA vectors using Daudi cells

(Method 1) Daudi cells (Ralph, P., et al., J. Exp. Med., 143, 1528-1533, 1976), as a cell strain derived from B cell, were cultured in RPMI-1640 medium (Merck, #R8758) containing 20% fetal bovine serum, and seeded on a 24 well plate under the condition of 2.5 x 10⁵ cells/well/400 µL. After 24 hours, the pseudotyped vector produced in Example 5 was diluted with an RPMI-1640 medium so as to have MOI = 1, and 100 µL of the diluted vector was added thereto. The cells were cultured for another 2 days, and then photographed with a fluorescence microscope (BZ-X800, Keyence Co.) to count the number of EGFP-positive cells. As a fluorescence filter, an OP-87763 BZX filter GFP (excitation wavelength 470/40, absorption wavelength 525/50, Keyence Corporation) was used.

(Method 2) Daudi cells were cultured in the same manner as in the method 1, and seeded on a 24 well plate under the condition of 1.5 x 10⁵ cells/well/400 µL. After 24 hours, the pseudotyped vector produced in Example 5 was diluted with an RPMI-1640 medium so as to be MOI (Multiplicity of Infection) = 3, and 100 µL of the diluted vector was added thereto. The cells were cultured for another 3 days, then fixed with 10 fold diluted 37% Formaldehyde (FUJIFILM Wako Pure Chemical Corporation) at room temperature for 10 minutes, and the number of EGFP positive cells was counted by Flow Cytometer (FCM) (BD LSRFortessa Cell Analyzer, BD Biosciences, Inc.). FCM was set such that excitation was a 488 nm laser, a detection system was a 515 - 545 nm bandpass filter (GFP-A), and a fraction having a signal intensity of 5 x 10³ or more in which a signal could not be detected in uninfected cells was determined to be EGFP-positive (FIG. 6).

### Example 8

### Comparison of production amounts of pseudotyped stealth RNA vectors by various combinations of chimeric F protein of Measles virus and chimeric H protein of Measles virus

Using plasmids pMS1 to pMS16 expressing a chimeric F protein of Measles virus and a chimeric H protein of Measles virus in various combinations, pseudotyped stealth RNA vectors were produced by the method described in Example 5, and the infectivity titer of vector particles released into the culture supernatant of BHK-21 cells was measured using Vero cells by the method described in Example 6. The stealth RNA vector can be concentrated by high-speed centrifugation, but considering that the stealth RNA vector can be concentrated about 30 times to a practical concentration of 1 x 10⁷ CIU/mL, the production capability was evaluated on the criterion of having production capability of 3 x 10⁵ CIU/mL or more. As a result, when four plasmids carrying MeV/SeV F#2 genes (pMS9, pMS10, pMS11, pMS12) or one plasmid carrying MeV Fdel30 genes (pMS14) were used, this criterion was satisfied (FIG. 7).

### Example 9

Comparison of ability of pseudotyped stealth RNA vectors to transfer genes into Daudi cells with various combinations of chimeric F protein of Measles virus and chimeric H protein of Measles virus

Next, the ability of the vector produced using each of the five plasmids selected in Example 8 to transfer genes into Daudi cells, which are suspension cell strains derived from blood was evaluated by the method described in the method 1 of Example 7.

A stealth RNA vector having an outer membrane glycoprotein of the Sendai virus for comparing the activities was produced by the following method. BHK-21/SRV-EGFP cells were seeded on a 12 well plate at 1 x 10⁵ cells/well/800 µL, and cultured for 1 day. The next day, 1.6 µg of the plasmid pS1 produced in Example 3, 0.8 µg of Plasmid D, 0.024 µg of pCMV-Furin (Patent Document 4, Example 6), 1.6 µL of a Lipofectamine PLUS reagent (Thermo Fisher Scientific Inc.), and 4 µL of Lipofectamine LTX (Thermo Fisher Scientific Inc.) were mixed in 100 µL of an Opti-MEM medium (Thermo Fisher Scientific Inc.), and the mixture was reacted at room temperature for 10 minutes. The medium containing this DNA was added to BHK-21/SRV-EGFP cells, and a DMEM medium containing 0.7 mL of 10% fetal bovine serum was added, and the cells were cultured for another 1 day. Next, the medium was replaced with a DMEM medium containing fresh 10% fetal bovine serum, the culture temperature was lowered to 32°C, and the cells were cultured for another 3 days to recover a culture supernatant containing a stealth RNA vector having the outer membrane glycoprotein of a Sendai virus. The collected supernatant was filtered through a 0.45 µm filter and then stored at 4°C. When it was necessary to store the supernatant for 2 days or more, the supernatant was cryopreserved at -80°C.

Although vectors of the same number of particles were added based on the infectivity evaluated in Vero cells, there was a large difference in gene transfer ability into Daudi cells. The Daudi cells are derived from B cells, and have low sensitivity to the Sendai virus, and low gene transfer activity by a stealth RNA vector having an outer membrane glycoprotein of the Sendai virus. Meanwhile, among the vectors produced using the five plasmids (pMS9, pMS10, pMS11, pMS12, pMS14) evaluated in this study, only one type (vector produced using pMS 10) reproducibly exhibited the gene transfer activity exceeding that of stealth RNA vectors having outer membrane glycoproteins of the Sendai virus (Table 1). This suggested that gene transfer into blood cells such as Daudi cells is partially different from the mechanism of gene transfer into Vero cells. Since one of the main objective of the present invention is "Efficient gene transfer into lymphocytes derived from peripheral blood such as B cells, CD4 positive T cells, and CD8 positive T cells, and immortalized cells derived from these cells", the following analysis was carried out based on the vector produced using pMS10.

The comparison of the efficiencies of gene transfer into Daudi cells using the pseudotyped stealth RNA vector is shown in Table 1 below.

**[Table 1]**

| Plasmid Name | cDNA #1 | cDNA #2 | Gene Delivery to Daudi cells (% of EGFP (+) cells) | |
|---|---|---|---|---|
| | | | Exp.1 | Exp.2 |
| pMS9 | MeV/SeV F #2 | MeV H | 0 | N.A |
| pMS10 | MeV/SeV F #2 | MeV/SeV H #1 | 20 | 20 |
| pMS11 | MeV/SeV F #2 | MeV/SeV H #2 | 2 | 1 |
| pMS12 | MeV/SeV F #2 | MeV Hdel18 | 2 | 2 |
| pMS14 | MeV Fdel30 | MeV/SeV H #1 | 10 | 3 |
| pS1 | SeV F | SeV HN | 5 | 6 |

### Example 10

### Comparison of chimeric F protein of Measles virus Edmonston strain and chimeric F protein of Measles virus wild-type strain in production of pseudotyped vector

Comparing F proteins of Measles virus Edmonston strain (vaccine strain adapted to Vero cells) and IC-B strain (wild-type strain, Takeuchi, K., et al., Virus Genes, 20, 253-257, 2000) isolated using B95a cells derived from Marmoset blood, the F protein of Edmonston strain has a structure in which 3 amino acid residues are longer on the N-terminal side due to 1-base mutation (FIG. 9). In order to examine whether this difference affects the production capability of the pseudotyped vector, cDNA (SEQ ID NO: 24) encoding MeV/SeV F#2 (SEQ ID NO: 34) of the IC-B strain was synthesized by the method described in Example 1, and expression plasmid pMS17 was produced by the method described in Example 3. Next, pseudotyped vectors were produced by the method described in Example 5 using pMS10 and pMS17, and the gene transfer activity of the vector in the culture supernatant was evaluated using Vero cells by the method described in Example 6 (Table 2). As a result, it was found that 1.65-times more vectors could be produced when pMS17 was used than when pMS10 was used, and further analysis was carried out using the pseudotyped vector produced using pMS17.

The comparison of the production amounts of the pseudotyped stealth RNA vector using the F protein of the Edmonston strain and the F protein of the IC-B strain is shown in Table 2 below.

**[Table 2]**

| Plasmid Name | cDNA #1 | cDNA #2 | CIU/mL (% of pMS10) |
|---|---|---|---|
| pMS10 | MeV/SeV F (Edmonston strain) #2 | MeV/SeV H #1 | 100 |
| pMS17 | MeV/SeV F (IC-B strain) #2 | MeV/SeV H #1 | 165 |

### Example 11

### Examination of gene transfer ability into Daudi cells of pseudotyped vector using chimeric F protein of Measles virus wild-type strain

As described in Example 9, the pseudotyped vector showing high gene transfer activity in Vero cells does not similarly have high gene transfer activity in blood cells. Therefore, the ability of the pseudotyped vector produced using pMS17 in Example 10 to infect Daudi cells was also examined. As a result of examination by the method described in the method 2 of Example 7, the vector produced using pMS17 could transfer genes into 59.2% Daudi cells, and high gene transfer activity could be reproduced (FIG. 8). Meanwhile, a pseudotyped vector (SRV (pMS1)) produced using an F protein and an H protein of a Measles virus as they are showed almost no gene transfer activity for Daudi cells (0.4%) although gene transfer into Vero cells could be performed (FIG. 8). From this, it was confirmed that a specific combination of a chimeric F protein and chimeric H protein is required, instead of using the envelope glycoprotein of the wild virus as it is, in order for the pseudotyped vector to have gene transfer activity for lymphocytic cells.

### Example 12

### Examination of gene transfer ability of pseudotyped vector using chimeric protein of Measles virus into CD19 positive primary cultured B cells derived from human peripheral blood

Next, the efficiency of gene transfer into primary cultured B cells was compared between the EGFP-carried pseudotyped stealth RNA vector produced using pMS17 described in Example 11 (hereinafter, abbreviated as SRV (Measles)) and the EGFP-carried stealth RNA vector produced using pS1 described in Example 9 (hereinafter, abbreviated as SRV (Sendai)).

The infectivity titers of SRV (Measles) and SRV (Sendai) were measured using Vero cells by the method described in Example 6. CD19 positive primary cultured B cells derived from human peripheral blood (Cell Application Inc., #6904-20a) were cultured in a B cell growth medium (2% of ImmunoCult-ACF Human B Cell Expansion Supplement (STEMCELL Technologies, Inc.) was added to ImmunoCult-XF T cell Expansion Medium (STEMCELL Technologies, Inc.)) for 7 days after thawing. As culture conditions, the cells were passaged once every two days at 2.5 x 10⁵ cells/mL.

On Day 7 of culture, cells were seeded on a 24 well plate in 2 x 10⁵ cells/0.4 mL B cell growth medium/well, 100 µL of SRV (Measles) and SRV (Sendai) diluted with the B cell growth medium so that MOI = 0.1 to 10 was satisfied, respectively, were added, and the cells were cultured for 24 hours at 37°C in the presence of 5% CO₂.

Next, the cells were transferred to a 1.5 mL tube, collected as a sediment by centrifugation at 400 x g for 5 minutes, washed once with the B cell growth medium, then seeded on a 12 well plate with 1 mL of the B cell growth medium/well, and cultured for 2 days at 37°C in the presence of 5% CO₂. Thereafter, the proportion of EGFP positive cells was measured by the method described in Example 7. As shown in FIG. 10 (left), SRV (Measles) could express EGFP by transferring genes into 78% of B cells at MOI = 1, 92% at MOI = 3, and 98% at MOI = 10. Meanwhile, SRV (Sendai) could only transfer genes into 7% cells at MOI = 1, 19% cells at an MOI = 3, and 41% cells at MOI = 10. From the above results, it was revealed that SRV (Measles) can transfer genes into CD19 positive B cells more efficiently than SRV (Sendai).

### Example 13

### Examination of gene transfer ability of pseudotyped vector using chimeric protein of Measles virus into CD4 positive primary cultured T cells derived from human peripheral blood

Next, SRV (Measles) and SRV (Sendai) described in Example 12 were used to compare gene transfer efficiencies into CD4-positive primary cultured T cells derived from human peripheral blood.

2 x 10⁵ cells/mL of CD4-positive primary cultured T cells derived from human peripheral blood (Astarte Biologics LIc, #1023) were thawed, and then cultured for 2 days in the presence of Dynabeads Human T-Activator CD3/CD28 (Beads: Cells = 1:1) ((Thermo Fisher Scientific, Inc.) using a T cell growth medium (Advanced RPMI medium (Thermo Fisher Scientific, Inc.), 10% fetal bovine serum, 1 x GlutaMAX (Thermo Fisher Scientific, Inc.), 30 units/mL Human recombinant IL-2 (Pepro Tech, #200-02)).

On the second day of culture, the cells were seeded on a 24 well plate in 2 x 10⁵ cells/0.4 mL T cell growth medium/well, 100 µL of SRV (Measles) and SRV (Sendai) diluted with the T cell growth medium so that MOI = 1 to 10 was satisfied, respectively, were added, and the cells were cultured for 24 hours at 37°C in the presence of 5% CO₂. Next, the cells were transferred to a 1.5 mL tube, collected as a sediment by centrifugation at 400 x g for 5 minutes, and then washed once with the T cell growth medium. Next, the cells were seeded on a 24 well plate in 0.5 mL of T cell growth medium/well, and cultured for 2 days at 37°C in the presence of 5% CO₂. Thereafter, the proportion of EGFP positive cells was measured by the method described in Example 7.

As shown in (middle) of FIG. 10, SRV (Measles) could express EGFP by transferring genes into 94% cells at MOI = 1 and 98% cells at MOI = 3. Meanwhile, SRV (Sendai) could only transfer genes into 34% cells at MOI = 1 and 52% cells at MOI = 3. From the above results, it was revealed that SRV (Measles) can transfer genes into CD4-positive primary cultured T cells derived from human peripheral blood more efficiently than SRV (Sendai).

### Example 14

### Examination of gene transfer ability of pseudotyped vector using chimeric protein of Measles virus into CD8 positive primary cultured T cells derived from human peripheral blood

Next, SRV (Measles) and SRV (Sendai) described in Examples 12 and 13 were used to compare gene transfer efficiencies into CD8 positive primary cultured T cells derived from human peripheral blood.

2 x 10⁵ cells/mL of CD8 positive primary cultured T cells derived from human peripheral blood (Lonza Group Ltd, #2W-300) were thawed, and then cultured for 2 days in the presence of Dynabeads Human T-Activator CD3/CD28 (Beads: Cells = 1:1) ((Thermo Fisher Scientific, Inc.) using a T cell growth medium (Advanced RPMI medium (Thermo Fisher Scientific, Inc.), 10% fetal bovine serum, 1 x GlutaMAX (Thermo Fisher Scientific, Inc.), 30 units/mL human recombinant IL-2 (Pepro Tech, #200-02)).

On the second day of culture, the cells were seeded on a 24 well plate in 2 x 10⁵ cells/0.4 mL T cell growth medium/well, 100 µL of SRV (Measles) and SRV (Sendai) diluted with the T cell growth medium so that MOI = 1 to 10 was satisfied, respectively, were added, and the cells were cultured for 24 hours at 37°C in the presence of 5% CO₂. Next, the cells were transferred to a 1.5 mL tube, collected as a sediment by centrifugation at 400 x g for 5 minutes, and then washed once with the T cell growth medium. Next, the cells were seeded on a 24 well plate in 0.5 mL of T cell growth medium/well, and cultured for 2 days at 37°C in the presence of 5% CO₂. Thereafter, the proportion of EGFP positive cells was measured by the method described in Example 7.

As shown in FIG. 10 (right), SRV (Measles) could express EGFP by transferring genes into 80% cells at MOI = 1 and 93% cells at MOI = 3. Meanwhile, SRV (Sendai) could only transfer genes into 33% cells at MOI = 1 and 54% cells at MOI = 3. From the above results, it was revealed that SRV (Measles) can transfer genes into CD8-positive primary cultured T cells derived from human peripheral blood more efficiently than SRV (Sendai).

### Example 15

### Production of Sendai virus vector pseudotyped with chimeric F protein of Measles virus and chimeric H protein of Measles virus

In Examples 5 to 14, using genomic RNA having an artificial base sequence optimized for human cells described in Example 6 of Patent Document 4 and a stealth RNA vector (SRV) having an RNA-dependent RNA polymerase of the Genus *Respirovirus* of *Paramyxoviridae* as materials, means for modifying the host cell specificity of "SRV having F protein and HN protein of Sendai virus in outer membrane" described in Patent Document 4 by outer membrane glycoproteins (F and H) of the Genus *Morbillivirus* of *Paramyxoviridae* were described.

As described above, the application range of the technology for improving the host cell specificity using the outer membrane glycoprotein of the morbillivirus is not limited to SRV, and all RNA virus vectors derived from the Genus *Paramyxoviridae* excluding the Genus *Morbillivirus* are included. In Example 15, a method for producing a pseudotyped vector using an outer membrane glycoprotein of a morbillivirus is shown, using a vector based on a Sendai virus (hereinafter, referred to as SeV) contained in Genus *Respirovirus* of *Paramyxoviridae* as a representative example.

By inserting the cRNA of any exogenous gene into the complete genomic RNA of the SeV, an SeV vector having autonomous replication ability can be produced (WO 97/16538, Hasan, M.K., et al., J. Gen. Virol., 78, 2813-2820, 1997). However, it is desirable that the vector used in industrial applications has lost the autonomous replication ability in order to ensure safety. The formation of SeV vector particles requires three types of proteins: an M protein present on the back side of the virus outer membrane, an F protein inducing fusion between the virus outer membrane and the cell membrane, and an HN protein responsible for binding between the virus outer membrane and the cell membrane. When any of these proteins is deleted, the autonomous replication ability is deficient or significantly reduced (Non-Patent Document 2). These proteins are encoded by an M gene, an F gene, and an HN gene on virus genomic RNA. By deleting one (Li, H-O., et al., J. Virology, 74, 6564-6569, 2000), two (WO 00/70070, Inoue, M., et al., J. Gene Med., 6, 1069-1081, 2004), or all three (Patent Document 1, Non-Patent Document 2, Yoshizaki, M., et al., J. Gene Med., 8, 1121-1159, 2006) of these three genes, it is possible to produce a vector in which the autonomous replication ability is deleted or significantly reduced. Therefore, whether pseudotyped vectors corresponding to each of the three types of vectors can be produced was examined.

<1> First, four SeV vectors in which one, two, or all three of an M gene, an F gene, and an HN gene were deleted were produced by the method described in Patent Document 1. In these vectors, 4 to 3 cDNAs of the following combinations are carried in order from the 3' side of the genomic RNA (FIG. 11).

### cDNAs carried on SeV vector A

(1) cDNA encoding M protein of SeV
(2) cDNA encoding chimeric protein MeV/SeV H#1 shown in FIG. 3
(3) cDNA encoding Kusabira Orange (Karasawa, S., et al., Biochem. J., 381, 307-312, 2004) derived from sea anemone
(4) cDNA encoding Blasticidin S deaminase (Kimura, M., et al., Biochim. Biophys. Acta, 1219, 653-659, 199432) derived from Actinomycetes

The base sequence of cDNA complementary to the sequence from the transcription termination signal of a P gene to the transcription initiation signal of an L gene, which includes the sequences of (1) to (4), is shown in SEQ ID NO: 25.

### cDNAs carried on SeV vector B

(1) cDNA encoding M protein of SeV
(2) cDNA encoding chimeric protein MeV/SeV F#2 shown in FIG. 2
(3) cDNA encoding Kusabira Orange derived from sea anemone
(4) cDNA encoding Blasticidin S deaminase derived from Actinomycetes

The base sequence of cDNA complementary to the sequence from the transcription termination signal of a P gene to the transcription initiation signal of an L gene, which includes the sequences of (1) to (4), is shown in SEQ ID NO: 26.

### cDNAs carried on SeV vector C

(1) cDNA encoding M protein of SeV
(2) cDNA encoding Kusabira Orange derived from sea anemone
(3) cDNA encoding Blasticidin S deaminase derived from Actinomycetes

The base sequence of cDNA complementary to the sequence from the transcription termination signal of a P gene to the transcription initiation signal of an L gene, which includes the sequences of (1) to (3), is shown in SEQ ID NO: 27.

### cDNAs carried on SeV vector D

(1) cDNA encoding Luciferase (RlucCP) (Promega Corp. GenBank #AY738228) derived from Cypridina
(2) cDNA encoding secreted Alkaline Phosphatase derived from human (Berger, J., et al., Gene, 66, 1-10, 1988)
(3) cDNA encoding Kusabira Orange derived from sea anemone
(4) cDNA encoding Blasticidin S deaminase derived from Actinomycetes

The base sequence of cDNA complementary to the sequence from the transcription termination signal of a P gene to the transcription initiation signal of an L gene, which includes the sequences of (1) to (4), is shown in SEQ ID NO: 28.

<2> An SeV vector A corresponds to a vector in which only an F gene is deleted (Li, H-O., et al., J. Virology, 74, 6564-6569, 2000), contains an M gene, and an F gene is deleted. An HN gene is substituted with MeV/SeV H#1 gene. An SeV vector B corresponds to a vector in which only an HN gene is deleted, and includes an M gene. An F gene is substituted with an MeV/SeV F#2 gene, and the HN gene is deleted. An SeV vector C corresponds to a vector in which two of an F gene and an HN gene are deleted (WO 00/70070), and contains an M gene. An F gene and an HN gene are deleted. An SeV vector D corresponds to a vector in which all of an M gene, an F gene, and an HN gene are deleted (Patent Document 1, Non-Patent Document 2, Yoshizaki, M., et al., J. Gene Med., 8, 1121-1159, 2006). The four SeV vectors described above were reconstituted from cDNA by the method described in Patent Document 1, and selected in a DMEM medium containing 10 µg/mL Blasticidin S (FUJIFILM Wako Pure Chemical Corporation) to establish BHK-21 cell strains containing the respective vectors (FIG. 11).
<3> Next, plasmid DNA for complementing the deleted gene was produced. First, the Plasmid_#4 described in Example 3 was cleaved with restriction enzymes BsrGI and XhoI, and a DNA fragment obtained by cleaving the MeV/SeV H#1 cDNA described in Example 2 with restriction enzymes Acc65I and XhoI was inserted to produce MeV/SeV H#1 gene expression plasmid DNA (Plasmid E). The Plasmid_#4 described in Example 3 was cleaved with the restriction enzymes BsrGI and XhoI, and a DNA fragment obtained by cleaving the MeV/SeV F#2 cDNA described in Example 1 with the restriction enzymes Acc65I and XhoI was inserted to produce MeV/SeV F#2 gene expression plasmid DNA (Plasmid F). Plasmid D expressing the M gene of a Sendai virus is described in Example 4. Plasmid pMS17 that simultaneously expresses MeV/SeV F#2 gene and MeV/SeV H#1 gene is described in Example 10.

The above plasmid DNA was purified by a cesium chloride density gradient method and a phenol extraction/chloroform extraction/ethanol precipitation method, then dissolved in a TE buffer (10 mM Tris-HCl, 1 mM EDTA, pH = 8), and stored at 4°C.

<4> Next, the following DNA was transferred into BHK-21 cells containing the SeV vector of the above <2> by the method described in Example 5.
BHK-21 cells containing SeV vector A; Plasmid F
BHK-21 cells containing SeV vector B; Plasmid E
BHK-21 cells containing SeV vector C; pMS17
BHK-21 cells containing SeV vector D; pMS17 + Plasmid D

A medium containing DNA was added to BHK-21 cells and the cells were cultured for 1 day. The medium was then replaced with a DMEM medium containing 10% fetal bovine serum to lower the culture temperature to 32°C. The cells were cultured for another 3 days to recover a culture supernatant containing a pseudotyped Sendai virus vector. The recovered supernatant was filtered through a 0.45 µm filter, and then the gene transfer activity was evaluated by the method described in Example 6. As a result, pseudotyped SeV vectors of 2.7 x 10⁵ CIU/mL or more could be produced in any combination.

### Example 16

### Establishment of iPS cells from B cells using stealth RNA vector pseudotyped with chimeric protein of Measles virus

Next, the efficiencies of establishing induced pluripotent stem cells (iPS cells) from primary cultured B cells derived from human peripheral blood were compared using a stealth RNA vector (hereinafter, abbreviated as SRV-iPSC (Sendai))carrying four reprogramming factors OCT4, SOX2, KLF4, and c-MYC, prepared using pS1 described in Example 9, and a pseudotyped vector (hereinafter, abbreviated as SRV-iPSC (Measles)) similarly carrying four reprogramming factors, prepared using pMS17 described in Example 10.

OCT4 cDNA (GenBank #NM_002701.4, SEQ ID NO: 29), SOX2 cDNA (GenBank #NM_003106.2, SEQ ID NO: 30), KLF4 cDNA (GenBank #NM_004235.4, SEQ ID NO: 31), and c-MYC cDNA (GenBank #NM_002467.3, SEQ ID NO: 32) were synthesized by adding GGTACCACC on the 5' side and CTCGAG on the 3' side of a coding region. Next, a stealth RNA vector (SRV-iPSC vector) in which these four cDNAs were carried in the order of OCT4, KLF4, SOX2, and c-MYC from the genome 3' side was produced by the method described in Patent Document 4.

BHK-21 cells were infected with SRV-iPSC vectors at MOI = 3 and 24 hours later pMS17 or pS1 was transferred by the methods described in Example 5. A medium containing DNA was added to BHK-21 cells and cultured for 1 day, and then replaced with a fresh DMEM medium containing 10% fetal bovine serum to reduce the culture temperature to 32°C.

The cells were cultured for another 3 days to collect culture supernatants containing SRV-iPSC (Sendai) and SRV-iPSC (Measles). The recovered supernatant was filtered through a 0.45 µm filter, and then the gene transfer activity was evaluated by the method described in Example 6.

CD19 positive primary cultured B cells derived from human peripheral blood (Cell Application Inc., #6904-20a) were thawed, and then cultured for 7 days by the method described in Example 12. On Day 7 of culture, the cells were seeded on a 24 well plate in 1 x 10⁵ cells/0.4 mL B cell growth medium/well, 100 µL of SRV (Measles) and SRV (Sendai) diluted with the B cell growth medium so that MOI = 3 was satisfied, respectively, were added, and the cells were cultured for 24 hours at 37°C in the presence of 5% CO₂.

Next, the cells were collected as a sediment by centrifugation at 300 x g for 5 minutes. The cells were washed once with 0.8 mL of a B cell growth medium and collected as a sediment by centrifugation at 300 x g for 5 minutes. 1 x 10⁴ cells were suspended in 0.4 mL of a B cell growth medium, seeded on a 24 well plate coated with iMatrix-511 (Nippi, Inc.) 0.9 µg/well (Day 0), and cultured at 37°C in the presence of 5% CO₂.

0.27 mL of an iPS cell culture medium StemFit AK02 (Ajinomoto Co., Inc.) was added on Day 1, Day 3, and Day 5, and the medium was replaced with a new StemFit AK02 medium on Day 7. Every other day, the medium was replaced with the new StemFit AK02 medium, and the culture was continued for another 5 days.

The iPS cells were immunostained with a TRA-1-60 antibody by the method described in Patent Document 2 on Day 12, and the number of TRA-1-60 positive cell colonies, which are an index of iPS cells, was measured under a fluorescence microscope. As a result, when the SRV-iPSC (Measles) vector was used, iPS cells could be induced with a high efficiency of 1.61%, whereas the reprogramming efficiency with the SRV-iPSC (Sendai) vector was 0.19% (FIG. 12). The number of B cells contained in 1 µL of adult peripheral blood is 250 to 1000, and when the reprogramming efficiency is increased to 1% or more, iPS cells can be established from B cells derived from 1 µL of peripheral blood. At present, induction of iPS cells from peripheral blood by an automated method has been studied in the field of regenerative medicine, and a pseudotyped stealth RNA vector is considered to be extremely effective for such industrial applications.

### Example 17

### Comparison of chimeric H protein of Measles virus Edmonston strain and chimeric H protein of Measles virus wild-type strain in production of pseudotyped vector

As shown in Example 10, in the production of the pseudotyped vector, when the case of using the chimeric F protein of the measles virus Edmonston strain (vaccine strain adapted to Vero cells) and the case of using the chimeric F protein of the IC-B strain (wild-type strain, Takeuchi, K., et al., Virus Genes, 20, 253-257, 2000) isolated using the B95a cells derived from the marmoset blood are compared, the production amount of the vector is improved using the chimeric F protein of the IC-B strain. As described above, the production of the pseudotyped vector is affected by the properties of the protein of Measles virus used as a material. Therefore, in the production of the chimeric H protein, the case of using the chimeric H protein of the Measles virus Edmonston strain and the case of using the chimeric H protein of the Measles virus IC-B strain were compared.

The H protein of the Measles virus Edmonston strain and the H protein of the Measles virus IC-B strain both consist of 617 amino acid residues, and there is a difference of 18 amino acid residues in total. The 173 amino acid residues on the N-terminal side are the same. The Edmonston strain MeV H protein binds to CD46 and SLAM on the cell surface. Meanwhile, there is a functional difference that the IC-B strain MeV H protein binds to SLAM on the cell surface but cannot bind to CD46 (Tatsuo, H., et al., Nature, 406, 893-897, 2000). Therefore, a cDNA (SEQ ID NO: 50) encoding the H protein MeV (IC-B)/SeV H#1 (SEQ ID NO: 51) of the modified Measles virus IC-B strain, in which the N-terminal 58 amino acid residues were substituted with the N-terminal 60 amino acid residues of the HN protein of the Sendai virus Z strain, was synthesized by the method described in Example 2.

Next, a plasmid vector expressing a chimeric F protein and a chimeric H protein was produced according to the method described in Example 3. Plasmid A was cleaved with restriction enzymes BsrGI and XhoI, and a DNA fragment obtained by cleaving a cDNA encoding MeV/SeV F#2 (SEQ ID NO: 34) of the IC-B strain described in Example 10 with restriction enzymes Acc65I and XhoI was inserted to produce Plasmid B (FIG. 4; In the figure, cDNA #1 represents a cDNA insert encoding a chimeric F protein). Furthermore, plasmid B was cleaved with the restriction enzymes Acc65I and SalI, and a DNA fragment obtained by cleaving a cDNA encoding MeV (IC-B)/SeV H#1 of the Measles virus IC-B strain described in Example 17 (SEQ ID NO: 51) with the restriction enzymes Acc65I and XhoI was inserted to produce pMS18 corresponding to plasmid C in FIG. 4. A large amount of pMS18 was prepared using E. coli NEB Stable strain (New England BioLab Inc.), and purified by a cesium chloride density gradient method and s phenol extraction-chloroform extraction-ethanol precipitation method, then dissolved in a TE buffer (10 mM Tris-HCl, 1 mM EDTA, pH = 8), and stored at 4°C.

Next, pseudotyped vectors were produced by the method described in Example 5 using pMS17 and pMS18, and the gene transfer activity of the vector in the culture supernatant was evaluated by the method described in Example 6. As cells for evaluation, Vero cells (CD46 positive, SLAM negative) and Vero/SLAM cells (CD46 positive, SLAM positive) (JCRB Cell Bank, #JCRB1809) produced by transferring a vector expressing SLAM into Vero cells were used (Table 3). As a result, in the evaluation system using Vero/SLAM cells, when pMS18 was used, a pseudotyped vector having 36% of the gene transfer activity when pMS17 was used was produced. Meanwhile, when the same cell supernatant was evaluated using SLAM-negative Vero cells, and pMS18 was used, the gene transfer activity was only 0.17% of that when pMS17 was used (Table 3).

From the above results, it became clear that when H protein MeV (IC-B)/SeV H#1 of the Measles virus IC-B strain is used, a pseudotyped vector reflecting the property of the IC-B strain that SLAM-positive cells can be infected but SLAM-negative/CD46 positive cells cannot be infected is obtained. Meanwhile, when SLAM-positive cells were used, the modified H protein MeV (IC-B)/SeV H#1 could be used to produce a pseudotyped vector having gene transfer activity close to that when the modified MeV/SeV H#1 was used.

The comparison of the production amounts of the pseudotyped stealth RNA vector using the H protein of the Edmonston strain and the H protein of the IC-B strain is shown in Table 3 below.

**[Table 3]**

| Plasmid Name | cDNA #1 | cDNA #2 | Titer with Vero/SLAM cells (% of pMS17) | Titer with Vero cells (% of pMS17) |
|---|---|---|---|---|
| pMS17 | MeV/SeV F (IC-B strain) chimera #2 | MeV/SeV H (Edmonston strain) chimera #1 | 100 | 100 |
| pMS18 | MeV/SeV F (IC-B strain) chimera #2 | MeV/SeV H (IC-B strain) chimera #1 | 36 | 0.17 |

### Example 18

Examination of gene transfer ability into Raji cells of pseudotyped vector using chimeric F protein of Measles virus wild-type strain and chimeric H protein of Measles virus wild-type strain

Next, for the pseudotyped vector produced using pMS18 in Example 17, the infectivity to cell strain Raji cells derived from human B cell (Epstein, M. A., J. Natl. Cancer Inst., 37, 547-559 (1966)) was examined according to the method 2 of Example 7. As a comparative object, a stealth RNA vector having the outer membrane glycoprotein of the Sendai virus produced using pS1 in Example 9 was used.

Raji cells (JCRB Cell Bank, #JCRB9012) were cultured in an RPMI-1640 medium (Merck, #R8758) containing 20% fetal bovine serum, then seeded on a 24 well plate under the condition of 2.5 x 10⁵ cells/well/400 µL, and the pseudotyped vector produced using pMS18 in Example 17 was diluted with an RPMI-1640 medium so that MOI (Multiplicity of Infection) = 1 was set with the gene transfer activity measured using Vero/SLAM cells, added in an amount of 100 µL, and cultured for 1 day. Next, the cells were transferred to a 1.5 mL tube, collected as a sediment by centrifugation at 400 x g for 5 minutes, and washed twice with Dulbecco's Phosphate Buffered Saline (Merck, D8662). Next, the cells were suspended in 500 µL of an RPMI-1640 medium containing 20% fetal bovine serum and seeded on a 24 well plate, cultured for 2 days, then fixed with 37% Formaldehyde (FUJIFILM Wako Pure Chemical Corporation) diluted 10 times at room temperature for 10 minutes, and the number of EGFP-positive cells was measured by a Flow Cytometer (FCM) (BD LSRFortessa Cell Analyzer, BD Biosciences, Inc.). The FCM was set such that excitation was a 488 nm laser, a detection system was a 515 - 545 nm bandpass filter (GFP-A), and a fraction having a signal intensity of 5 x 10³ or more in which a signal could not be detected in uninfected cells was determined to be EGFP-positive.

As a result, the pseudotyped vector produced using pMS18 was able to express EGFP by transferring the genes into 43.2% Raji cells under the condition of MOI = 1. Meanwhile, a stealth RNA vector having an outer membrane glycoprotein of the Sendai virus expressed EGFP by transferring the genes into 15.9% of Raji cells under the condition of MOI = 1. From the above results, it was confirmed that the pseudotyped vector using the chimeric F protein of the Measles virus wild-type strain and the chimeric H protein of the Measles virus wild-type strain can more efficiently transfer genes into Raji cells derived from human B cells than the stealth RNA vector having the outer membrane glycoprotein of the Sendai virus.

## Claims

1. A chimeric F protein of a paramyxovirus, which is any of the following (1) to (8):
(1) a polypeptide composed of an amino acid sequence encoded by a base sequence of SEQ ID NO: 3,
(2) a polypeptide comprising an amino acid sequence encoded by a base sequence of SEQ ID NO: 3,
(3) a polypeptide composed of an amino acid sequence encoded by a base sequence of SEQ ID NO: 24,
(4) a polypeptide comprising an amino acid sequence encoded by a base sequence of SEQ ID NO: 24,
(5) a polypeptide composed of an amino acid sequence of SEQ ID NO: 33,
(6) a polypeptide comprising an amino acid sequence of SEQ ID NO: 33,
(7) a polypeptide composed of an amino acid sequence of SEQ ID NO: 34, and
(8) a polypeptide comprising an amino acid sequence of SEQ ID NO: 34.

2. A combination of proteins comprising the chimeric F protein of a paramyxovirus according to claim 1 and an H/HN chimeric protein of a paramyxovirus which is any of the following (1) to (4):
(1) a polypeptide composed of an amino acid sequence encoded by a base sequence of SEQ ID NO: 9,
(2) a polypeptide comprising an amino acid sequence encoded by base sequence of SEQ ID NO: 9,
(3) a polypeptide composed of an amino acid sequence of SEQ ID NO: 35, and
(4) a polypeptide comprising an amino acid sequence of SEQ ID NO: 35.

3. A vector capable of expressing a chimeric F protein of a paramyxovirus, the vector being any one of the following (1) to (4):
(1) a vector comprising a polynucleotide having a base sequence of SEQ ID NO: 3,
(2) a vector comprising a polynucleotide having a base sequence of SEQ ID NO: 24,
(3) a vector comprising a polynucleotide encoding a polypeptide having an amino acid sequence of SEQ ID NO: 33, and
(4) a vector comprising a polynucleotide encoding a polypeptide having an amino acid sequence of SEQ ID NO: 34.

4. The vector according to claim 3, further comprising any polynucleotide of the following (1) or (2):
(1) a polynucleotide having a base sequence of SEQ ID NO: 9, and
(2) a polynucleotide encoding a polypeptide having an amino acid sequence of SEQ ID NO: 35.

5. The vector according to claim 3, wherein the vector is a plasmid vector.

6. The vector according to claim 4, wherein the vector is a plasmid vector.

7. A combination of vectors comprising a vector capable of expressing a chimeric F protein of a paramyxovirus and a vector capable of expressing a chimeric H/HN protein of a paramyxovirus, including the vector according to claim 3 and a vector according to any one of the following (1) and (2):
(1) a vector comprising a polynucleotide having a base sequence of SEQ ID NO: 9, and
(2) a vector comprising a polynucleotide encoding a polypeptide having an amino acid sequence of SEQ ID NO: 35.

8. The combination of the vectors according to claim 7, wherein the combination is a combination of plasmid vectors.

9. A host cell transformed with the vector according to any of claims 3 to 6 or the combination of the vectors according to claim 7 or 8.

10. The transformed host cell according to claim 9, wherein the host cell is a eukaryotic cell.

11. A pseudotyped virus particle having a negative-sense single-stranded RNA genome comprising the chimeric F protein according to claim 1 or the combination of proteins according to claim 2, as an envelope protein.

12. The virus particle according to claim 11, wherein the negative-sense single-stranded RNA genome includes a cRNA sequence encoding exogenous protein(s).

13. A method for transferring a gene into a lymphocyte derived from human peripheral blood, the method comprising the process of contacting the lymphocyte derived from the human peripheral blood with the virus particle according to claim 12.
